# EUROPEAN PATENT SPECIFICATION

(11) **EP 3 129 552 B1**
(45) Date of publication and mention of the grant of the patent: **22.05.2019**
(21) Application number: 14888854.8
(22) Date of filing: 08.04.2014
(51) Int. Cl.: D21H 27/00, A47K 10/16, D04H 1/413, D04H 1/492, D21H 21/14, D21H 21/50, D21H 21/52

(54) **FLUSHABLE HYDROENTANGLED MOIST WIPE OR HYGIENE TISSUE**
SPÜLBARES WASSERSTRAHLVERFESTIGTES FEUCHTTUCH ODER HYGIENETUCH
CHIFFON OU TISSU D'HYGIÈNE HUMIDE HYDROLIÉ JETABLE DANS LES TOILETTES

(43) Date of publication of application: 15.02.2017
(73) Proprietor: Essity Hygiene and Health Aktiebolag, 405 03 Göteborg (SE)
(72) Inventor: STRÅLIN, Anders, 423 38 Torslanda (SE); AHONIEMI, Hannu, 405 03 Göteborg (SE); FINGAL, Lars, 405 03 Göteborg (SE); NIHLSTRAND, Anna, 405 03 Göteborg (SE)
(74) Representative: Valea AB
(86) International application number: PCT/SE2014/050433
(87) International publication number: WO 2015/156713

(56) References cited:
- EP-A1- 1 302 592
- WO-A1-2006/060816
- WO-A1-2011/046478
- WO-A1-2011/046478
- WO-A1-2012/050494
- WO-A1-2013/015735
- DE-B3-102010 009 134
- US-A- 4 755 421
- US-A1- 2003 100 240
- US-A1- 2010 143 646

## Description

### TECHNICAL FIELD

The present invention refers to a flushable hydroentangled wipe or hygiene tissue impregnated with a wetting composition, wherein the nonwoven material comprises cellulose pulp fibers and/or manmade staple fibers.

### BACKGROUND OF THE INVENTION

Pre-moistened wipes or hygiene tissue, are commonly used for cleansing different parts of the human body. Examples of specific uses are baby care, hand wiping, feminine care and toilet paper or a complement to toilet paper.

Since a long period of time often elapses from the time of manufacture of pre-moistened wipes until the time of use, they must have a sufficient structural integrity for their intended wiping function during such period. Adding a wet strength agent to the wipe will provide such wet integrity. However, especially when used as toilet paper, there is a strong desire that the wipe or tissue can be flushed in the sewer without causing problems with blocked pipes and filters. Wipes or tissue having a high wet strength will not disintegrate or break up into small fibre clumps when flushed in conventional household toilet systems, which may cause plugging of the drainage system.

Previously moist flushable pre-moistened wipes and toilet papers which were on the market were flushable due to their small size. They could move along the drainage and sewage pipes, but were not readily dispersible and could therefore cause problems with blocked pipes and filters. Nowadays disintegratable materials are available for use in flushable wipes and hygiene tissue.

US 4,755,421 discloses a hydroentangled disintegratable nonwoven fabric used as a flushable wet wipe. The fabric comprises a mixture of pulp fibers and staple length regenerated cellulose fibers. No binder is added.

US 2004/0013859 discloses a hydroentangled nonwoven web used as a wet wipe that is disintegrated with mild agitation in water. The nonwoven web comprises natural cellulose fibers and high crystallinity cellulose fibers, preferably lyocell fibers. A binder is also added, for example in the form of binder fibers. The nonwoven web is said to have high wet tensile strength but disintegrates or disperses with mild agitation in water, making it flushable in a sewer.

WO 2013/015735 discloses a hydroentangled moist wipe that is flushable. The wipe material comprises pulp fibers and poly(lactic acid) fibers having a length between 8 and 20 mm. The wipe is free from added binders and wet-strength agents.

JP 2008073357 discloses a hydroentangled wipe material that has water dissolving properties. The wipe material comprises hydrophilic fibers and fibers having a modified cross-section.

JP 2012057289 discloses a flat hollow rayon fiber with creases formed in the outer surface. These flat rayon fibers may be used in different kinds of products, wherein water-dispersible sheets are mentioned as one example.

There is however still a need for a moist wipe or hygiene tissue which has sufficient structural integrity for its intended wiping function but which is readily disintegratable when flushed in a sewer.

### SUMMARY OF THE INVENTION

The object of the present invention is therefore to provide a moist wipe or hygiene tissue having sufficient wet strength for its intended use but which is disintegrated when flushed in a sewer. This object has according to the invention been solved by the fact that the moist wipe or hygiene tissue comprises a hydraulically entangled nonwoven material impregnated with a wetting composition, said nonwoven material containing cellulose pulp fibers and/or manmade staple fibers, wherein the nonwoven material also comprises disintegration elements having a projected surface area between 2 and 50 mm² and an aspect ratio between 1 and 10.

These disintegrating elements will create spots in the nonwoven material, that weaken the material resulting in a material that disintegrates or disperses in water under mild agitation, such as present in a standard sewer.

The flushable wipe or hygiene tissue comprises between 0.5 and 2.5 weight% of said disintegration elements as calculated on the dry weight of the wipe or hygiene tissue.

Said disintegration elements may be of a material having a disintegration time as measured by French Standard NF Q 34-020 that differs from the nonwoven material.

Said disintegration elements may be of a material having a slower disintegration time as measured by French Standard NF Q 34-020 than the nonwoven material.

The disintegration elements are of a material selected from the following group: botanical elements, paper containing a wet-strength agent, nonwoven material, film material The disintegration elements may have an aspect ratio between 1 and 7.

The thickness of the disintegration elements may be between 50 µm and the thickness of the nonwoven material, preferably below 1 mm.

The disintegration elements may be embedded by fibers in the nonwoven material.

The disintegration elements may have a different colour or tint than the rest of the nonwoven material.

The disintegrating elements may have a decorative and/or informative shape, such as symbols, characters, logotypes and the like.

### BRIEF DESCRIPTION OF THE FIGURES

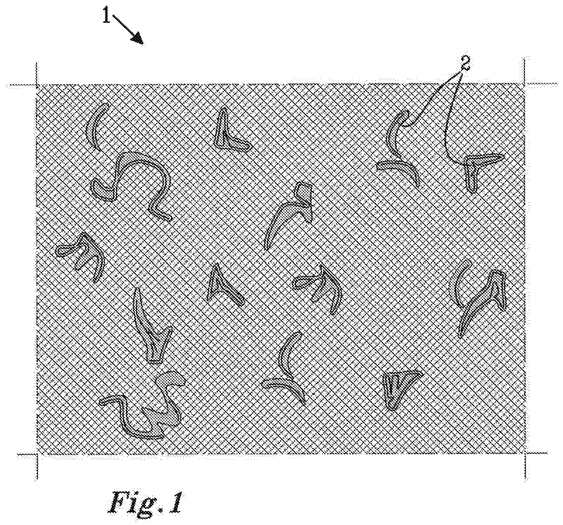

Fig. 1 is a schematic sketch of a hydroentangled material comprising disintegration elements according to the invention.
Fig. 2 a-h are schematic sketches of disintegration elements having different shapes and illustrate how the aspect ratio is measured.

### DETAILED DESCRIPTION OF PREFERRED EMBODIMENTS

A premoistened wipe or hygiene tissue according to the invention comprises a hydroentangled nonwoven material impregnated with a wetting composition. The wetting composition may contain a major proportion of water and other ingredients depending on the intended use. Wetting compositions useful in moist wipes and hygiene tissue are well-known in the art.

Hydroentangling or spunlacing is a technique for forming a nonwoven web introduced during the 1970'ies, see e g CA patent no. 841 938. The method involves forming a fibre web, which is either drylaid or wetlaid, after which the fibres are entangled by means of very fine water jets under high pressure. Several rows of water jets are directed against the fibre web, which is supported by a movable foraminous support or a perforated drum. In this process the fibres entangle with one another providing sufficient bonding strength to the fibrous web without the use of chemical bonding agents. The entangled fibrous web is then dried. The fibres that are used in the material can be natural fibres, especially cellulosic pulp fibres, manmade staple fibres, and mixtures of pulp fibres and staple fibres. Hydroentangled materials can be produced with high quality at a reasonable cost and they possess a high absorption capacity.

The wipe or hygiene tissue according to the invention may comprise a mixture of cellulosic pulp fibers and manmade fibers, preferably biodegradable manmade fibers such as regenerated cellulose fibres, e.g. viscose, rayon and lyocell, and/or poly(lactic acid) fibers. The length of these manmade fibres may be in the range of 4 to 20 mm. Other natural fibres than pulp fibres may also be included in the fibrous web, such as cotton fibres, sisal, hemp, ramie, flax etc. These natural fibres usually have a length of more than 4 mm.

Cellulose pulp fibres can be selected from any type of pulp and blends thereof. Preferably the pulp is characterized by being entirely natural cellulosic fibres and can include wood fibres as well as cotton. Preferred pulp fibres are softwood papermaking pulp, although hardwood pulp and non-wood pulp, such as hemp and sisal may be used. The length of pulp fibres may vary from less than 1 mm for hardwood pulp and recycled pulp, to up to 6 mm for certain types of softwood pulp. Pulp fibres are advantageous to use since they are inexpensive, readily available and absorbent.

A suitable amount of cellulose pulp fibers in the nonwoven material forming the moist wipe or hygiene tissue may be between 0 and 95% by weight cellulose pulp fibers, preferably between 50 and 95% by weight cellulose pulp fibers and more preferably between 70 and 95% by weight cellulose pulp fibers. A suitable amount of manmade fibers in the nonwoven material forming the moist wipe or hygiene tissue may be between 5 and 100% by weight manmade fibers, preferably between 5 and 50% by weight manmade fibers and more preferably between 5 and 30% by weight manmade fibers. The wipe or hygiene tissue may have a basis weight in the range 30 to 100 gsm, preferably 40 to 80 gsm, based on the dry weight of the material.

The fibers forming the nonwoven material according to the invention may be wetlaid, wherein a slurry comprising the fibres is wetlaid on a moving forming fabric by a headbox as in a conventional papermaking equipment. A special variant of wetlaying or wetforming is foamforming, wherein the fibres are dispersed in a foamed liquid containing water and a surfactant. Alternatively the fibers may be drylaid on a moving forming fabric to form a drylaid fibrous web which is subsequently hydroentangled..

In addition to the fibers the nonwoven material 1 comprises disintegration elements 2 in the form of flakes having a projected surface area between 2 mm² and 50 mm² and an aspect ratio L/D between 1 and 10, preferably between 1 and 7. The projected surface area may be measured by image analysis.

The aspect ratio is defined as the ratio of the length L to the width D of the disintegration element 2. The length L is defined as the longest straight line that can be drawn/found in the element. The width D is defined as the longest straight line that can be found/drawn in said element perpendicular to the line L. No parts of the lines L and D should cross the edge of the element, i.e. the full length of the lines L and D must be inside the element. In cases where two or more lines with the same length can be found (L1=L2=...Lx), the length L which generates the longest line D, i.e. resulting in the lowest L/D ratio, should be used.

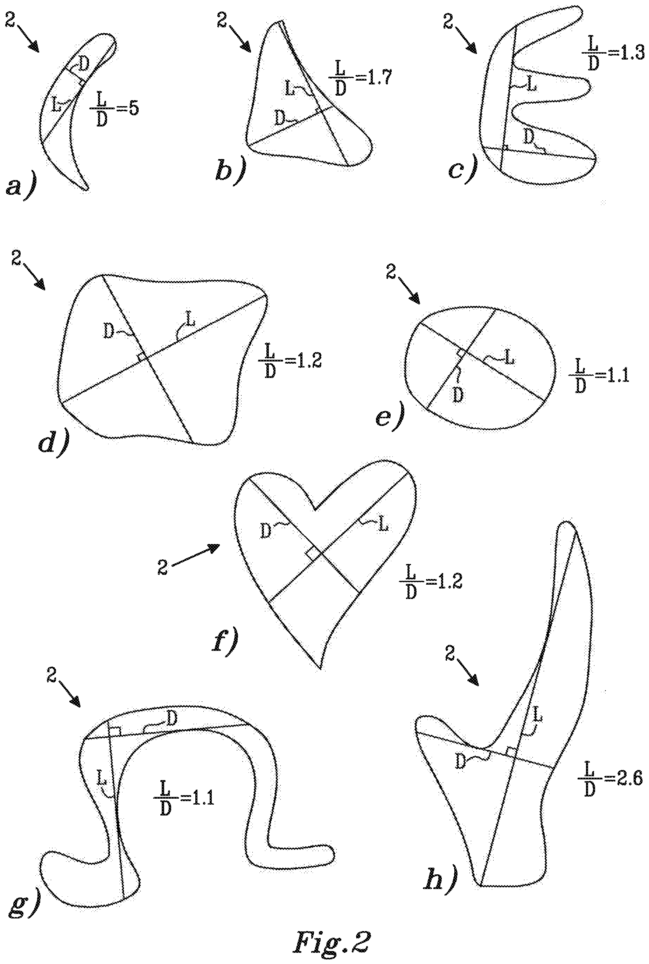

Fig. 2 a-h illustrate how the aspect ratio L/D is measured for disintegration elements 2having varying shapes.

The disintegration elements 2 are mixed with the fibers, for example in the slurry used in wetlaying.

The disintegration elements 2 in the form of flakes having the above mentioned proportions are different from fibers and will not easily entangle with the fibers during hydroentangling. They will rather form spots in the nonwoven material that will weaken the material, preferably in the cross direction, CD, thus making it more readily disintegratable when flushed in a sewer.

The disintegration elements 2 are contained in the nonwoven material 1 in an amount between 0.5% by weight and 2.5% by weight as calculated on the dry weight of the nonwoven material, i.e. excluding the wetting composition. The disintegration elements 2 are preferably distributed throughout the area of the nonwoven material 1.

The disintegrating elements 2 are of a material selected from: botanical elements, wet-strong paper, i.e. paper containing a wet-strength agent, nonwoven material, film material. Preferably the disintegrating elements 2 are of a biodegradable material. They should further be of a material that has a disintegration time as measured by French Standard NF Q 34-020 that differs from the nonwoven material as such, for example a slower disintegration time, such as at least 10% longer disintegration time than the nonwoven material. This will ensure that they will maintain their effect of weakening the nonwoven material until this has been disintegrated. The thickness of the disintegration elements should be at least 50 µm and not more than the thickness of the nonwoven material, preferably less than 1 mm.

Examples of botanical elements suited for use as disintegrating elements are petals, e g from marigold, lavender or similar flowers. Such botanical elements may have a colour or tint different from the nonwoven material as such. They may also have a fragrance and/or a skin care effect.

The disintegrating elements 2 may be formed of cut pieces of a sheet material, e g wet-strong paper, nonwoven or film material. These pieces may have a colour or tint different from the rest of the nonwoven material. They may also have a decorative and/or informative shape such as symbols, characters, logotypes and the like.

### DESCRIPTION OF TEST METHOD

The test method for measuring disintegration time is French Standard NF Q 34-020 August 1998. In addition of measuring the disintegration time we also measured the weight of the remaining lump of fibers (if any) in the test vessel. The test was performed in the following manner:
- Put the dried sample in a climate room 23°C, 50% humidity for minimum 4 hours;
- Weigh the sample before start;
- Stop the timer when the sample is disintegrated into pieces smaller than 1 cm² and record the disintegration time;
- Alt. 1: Start stirring again and let stir until 10 minutes have passed (including the initial disintegration time);
- Alt. 2: Omit the additional step of stirring 10 minutes;
- Pick up the remaining lump with a pair of tweezers;
- Put the lump in a 900 ml beaker filled with water up to 300 ml, wait 5 sec.;
- Pick up the lump with tweezers and let excess water drip off, wait 5 sec.;
- Put the lump in a second 900 ml beaker filled with water up to 300 ml, wait 5 sec.;
- Pick up the lump with tweezers and let excess water drip off, wait 5 sec.;
- Put the lump on a tissue paper to drain excess water;
- Put the lump in a climate chamber for 2h 80°C to dry;
- Put the dried lump in a climate room 23 °C, 50% humidity, for minimum 4h;
- Weigh the lump;
- Calculate the remaining weight of the dried lump as a percentage of the initial dry sample weight (dry weight of remaining lump / dry weight of sample) x 100 (%).

### EXAMPLES

Trials have been made on hydroentangled nonwoven materials containing disintegration elements in the form of marigold petals according to the invention and a reference sample containing no disintegration elements. The samples had the following composition.

Reference: 80 wt% cellulose pulp + 10 wt% lyocell fibers 12 mm + 10 wt% PLA:poly(lactic acid) fibers 12 mm. The basis weight was 60 gsm and the material was hydroentangled with 3 manifolds/jet strips on each sides of the web with 60 bars with entanglement nozzles hole diameter 115 µm with a pitch of 0.8 mm between holes. The sample size and number of folds was adapted to Samples 1-4 referred to below.
Sample 1: 79 wt% cellulose pulp + 10 wt% lyocell fibers 12 mm + 10 wt% PLA:poly(lactic acid) fibers 12 mm + 1 wt% marigold petals.. The basis weight was 60 gsm and the material was hydroentangled with 3 manifolds/jet strips on each side of the web with 60 bars with entanglement nozzles. Sample size was 10x25 cm, folded twice in longitudinal direction.
Sample 2: 79 wt% cellulose pulp + 10 wt% lyocell fibers 12 mm + 10 wt% PLA:poly(lactic acid) fibers 12 mm + 1 wt% marigold petals. The basis weight was 60 gsm and the material was hydroentangled with 3 manifolds/jet strips on each side of the web with 60 bars with entanglement nozzles. Sample size was 10x25 cm, folded once in longitudinal direction.
Sample 3: a) 79 wt% cellulose pulp + 10 wt% lyocell fibers 12 mm + 10 wt% PLA:poly(lactic acid) fibers 12 mm + 1 wt% marigold petals; b) 77 wt% cellulose pulp + 10 wt% lyocell fibers 12 mm + 10 wt% PLA:poly(lactic acid) fibers 12 mm + 3 wt% marigold petals. The basis weight was 60 gsm and the materials were hydroentangled with 3 manifolds/jet strips on each side of the web with 60 bars with entanglement nozzles. Sample size was 10x10 cm with no fold.
Sample 4: 77 wt% cellulose pulp + 10 wt% lyocell fibers 12 mm + 10 wt% PLA:poly(lactic acid) fibers 12 mm + 3 wt% marigold petals. The basis weight was 60 gsm and the material was hydroentangled with 3 manifolds/jet strips on each side of the web with 60 bars with entanglement nozzles. Sample size was 10x25 cm folded once in longitudinal direction.

Wet strength in water in CD according to SS-EN ISO 12625-5:2005 was measured for the different samples as well as disintegration time and weight of remaining lump were measured. The following results were obtained.

**Sample 1**

| | CD wet tensile strength¹ (N/m) | Basis weight ² (g/m2) | Disinteg ration time³ (sec) | Lump (wt-%) |
|---|---|---|---|---|
| Reference | 24,5 | 62,0 | 163 | Small, not measured |
| 1% Marigold | 24,4 | 63,4 | 139 | Small, not measured |
| Difference (Ref-1%): | -0,2% | | -14,7% | |

| | | | | |
|---|---|---|---|---|
| ¹ Average of 10 samples ² Average of 15 samples ³ Average of 8 samples | | | | |

**Sample 2**

| | CD wet tensile strength¹ (N/m) | Basis weight ² (g/m2) | Disinteg ration time² (sec) | Lump at 10min (Alt. 1) (wt-%) |
|---|---|---|---|---|
| Reference | 24,5 | 61,4 | 156 | 9,8 |
| 1% Marigold | 24,4 | 63,8 | 124 | 4,8 |
| Difference (Ref-1% Marigold): | -0,2% | | -20.5% | |

| | | | | |
|---|---|---|---|---|
| ¹ Average of 10 samples ² Average of 9 samples | | | | |

**Sample 3**

| | CD wet tensile strength¹ (N/m) | Basis weight¹ (g/m2) | Disinteg ration time¹ (sec) | Lump at disintegration time¹ (Alt. 2) (wt-%) |
|---|---|---|---|---|
| Reference | - | 64,1 | 107 | 9,5 |
| 1% Marigold | - | 64,1 | 80 | 4,1 |
| 3% Marigold | - | 64,2 | 119 | 19,9 |
| Difference (Ref-1% Marigold): | | | -24,8% | |
| Difference (Ref-3% Marigold): | | | +12,0% | |

| | | | | |
|---|---|---|---|---|
| ¹ Average of 5 samples | | | | |

**Sample 4**

| | CD wet tensile strength¹ (N/m) | Basis weight (g/m2) | Disinteg ration time (sec) | Lump at 10min (Alt. 1) (wt-%) |
|---|---|---|---|---|
| Reference | 35,3 | 64,6² | 151 ² | 2,1 ² |
| 3% Marigold | 26,9 | 63,9³ | 146 ³ | 3,9 ³ |
| Difference (Ref-3%): | -23,9% | | -2.8% | |

| | | | | |
|---|---|---|---|---|
| ¹ Average of 5 samples ² Average of 4 samples ³ Average of 9 samples | | | | |

It is interesting to note that a 1% admixture of marigold petals resulted in a significantly lower disintegration time as well as lump than for the reference, while the CD wet strength was maintained. A 3% admixture of marigold petals, however, resulted in an increased disintegration time and lump and a decrease in CD wet strength.

## Claims

1. A flushable wipe or hygiene tissue comprising a hydraulically entangled nonwoven material impregnated with a wetting composition, said nonwoven material (1) comprising cellulose pulp fibers and/or manmade staple fibers,
**characterized in that**
the nonwoven material comprises disintegration elements (2) having a projected surface area between 2 and 50 mm² and an aspect ratio (L/D) between 1 and 10, said flushable wipe or hygiene tissue comprises between 0.5 and 2.5 weight% of said disintegration elements (2) as calculated on the dry weight of the wipe or hygiene tissue, and
said disintegration elements (2) are of a material selected from the following group: botanical elements, paper containing a wet-strength agent, nonwoven material, film material.

2. A flushable wipe or hygiene tissue as claimed in claim 1, **characterized in that** said disintegration elements (2) are of a material having a disintegration time as measured by French Standard NF Q 34-020 that differs from the nonwoven material.

3. A flushable wipe or hygiene tissue as claimed in claim 2, **characterized in that** said disintegration elements (2) are of a material having a slower disintegration time as measured by French Standard NF Q 34-020 than the nonwoven material

4. A flushable wipe or hygiene tissue as claimed in any of the preceding claims, **characterized in that** the disintegration elements (2) have an aspect ratio between 1 and 7.

5. A flushable wipe or hygiene tissue as claimed in any of the preceding claims, **characterized in that** the thickness of the disintegration elements (2) is between 50 µm and the thickness of the nonwoven material, preferably below 1 mm.

6. A flushable wipe or hygiene tissue as claimed in any of the preceding claims, **characterized in that** disintegration elements (2) are embedded by fibers in the nonwoven material.

7. A flushable wipe or hygiene tissue as claimed in any of the preceding claims, **characterized in that** the disintegration elements (2) have a different colour or tint than the rest of the nonwoven material.

8. A flushable wipe or hygiene tissue as claimed in any of the preceding claims, **characterized in that** the disintegrating elements has a decorative and/or informative shape, such as symbols, characters, logotypes and the like.

## Patentansprüche

1. Spülbares Tuch oder Hygiene-Tissue, umfassend ein hydraulisch verfestigtes Vliesmaterial, das mit einer benetzenden Zusammensetzung imprägniert ist, wobei das Vliesmaterial (1) Cellulosepulpe-Fasern und/oder künstlich hergestellte Stapelfasern umfasst,
**dadurch gekennzeichnet, dass**
das Vliesmaterial Desintegrationselemente (2) mit einer projizierten Oberfläche zwischen 2 und 50 mm² und einem Seitenverhältnis (L/D) zwischen 1 und 10 umfasst, wobei das spülbare Tuch oder das Hygiene-Tissue zwischen 0,5 und 2,5 Gew.-% der Desintegrationselemente (2) umfasst, berechnet auf Grundlage des Trockengewichts des Tuchs oder des Hygiene-Tissues, und
wobei die Desintegrationselemente (2) aus einem Material sind, das ausgewählt ist aus der folgenden Gruppe: pflanzliche Elemente, Papier, das ein Nassverfestigungsmittel enthält, Vliesmaterial, Folienmaterial.

2. Spülbares Tuch oder Hygiene-Tissue gemäß Anspruch 1,
**dadurch gekennzeichnet, dass** das die Desintegrationselemente (2) aus einem Material mit einer Desintegrationszeit sind, welche sich von dem Vliesmaterial unterscheidet, gemessen mit dem französischen Standard NF Q 34-020.

3. Spülbares Tuch oder Hygiene-Tissue gemäß Anspruch 2,
**dadurch gekennzeichnet, dass** die Desintegrationselemente (2) aus einem Material sind, das eine langsamere Desintegrationszeit als das Vliesmaterial aufweist, gemessen mit dem französischen Standard NF Q 34-020.

4. Spülbares Tuch oder Hygiene-Tissue gemäß irgendeinem der vorstehenden Ansprüche, **dadurch gekennzeichnet, dass** die Desintegrationselemente (2) ein Seitenverhältnis zwischen 1 und 7 aufweisen.

5. Spülbares Tuch oder Hygiene-Tissue gemäß irgendeinem der vorstehenden Ansprüche, **dadurch gekennzeichnet, dass** die Dicke der Desintegrationselemente (2) zwischen 50 µm und der Dicke des Vliesmaterials liegt, bevorzugt unterhalb von 1 mm.

6. Spülbares Tuch oder Hygiene-Tissue gemäß irgendeinem der vorstehenden Ansprüche, **dadurch gekennzeichnet, dass** die Desintegrationselemente (2) durch Fasern in das Vliesmaterial eingebettet sind.

7. Spülbares Tuch oder Hygiene-Tissue gemäß irgendeinem der vorstehenden Ansprüche, **dadurch gekennzeichnet, dass** die Desintegrationselemente (2) eine(n) von dem Rest des Vliesmaterials verschiedene(n) Farbe oder Farbton aufweisen.

8. Spülbares Tuch oder Hygiene-Tissue gemäß irgendeinem der vorstehenden Ansprüche, **dadurch gekennzeichnet, dass** die Desintegrationselemente eine dekorative und/oder informative Form aufweisen, wie z.B. Symbole, Zeichen, Logos und dergleichen.

## Revendications

1. Lingette ou serviette hygiénique jetable dans les toilettes comprenant un matériau non tissé à enchevêtrement hydraulique imprégné par une composition humidifiante, ledit matériau non tissé (1) comprenant des fibres de pulpe de cellulose et/ou des fibres discontinues synthétiques,
**caractérisée en ce que**
le matériau non tissé comprend des éléments de dissolution (2) ayant une surface projetée comprise entre 2 et 50 mm² et un rapport de forme (L/D) compris entre 1 et 10, ladite lingette ou serviette hygiénique jetable dans les toilettes comprend entre 0,5 et 2,5% en poids desdits éléments de dissolution (2), tel que calculé selon le poids sec de la lingette ou de la serviette hygiénique, et
lesdits éléments de dissolution (2) sont en un matériau sélectionné à partir du groupe suivant : les éléments végétaux, du papier contenant un agent de résistance à l'état humide, un matériau non tissé, un matériau pelliculaire.

2. Lingette ou serviette hygiénique jetable dans les toilettes selon la revendication 1, **caractérisée en ce que** lesdits éléments de dissolution (2) sont en un matériau ayant une durée de dissolution, telle que mesurée selon la norme française NF Q 34-020, qui diffère du matériau non tissé.

3. Lingette ou serviette hygiénique jetable dans les toilettes selon la revendication 2, **caractérisée en ce que** lesdits éléments de dissolution (2) sont en un matériau ayant une durée de dissolution, telle que mesurée selon la norme française NF Q 34-020, plus faible que le matériau non tissé.

4. Lingette ou serviette hygiénique jetable dans les toilettes selon l'une quelconque des revendications précédentes, **caractérisée en ce que** lesdits éléments de dissolution (2) ont un rapport de forme (L/D) compris entre 1 et 7.

5. Lingette ou serviette hygiénique jetable dans les toilettes selon l'une quelconque des revendications précédentes, **caractérisée en ce que** l'épaisseur des éléments de dissolution (2) est comprise entre 50 µm et l'épaisseur du matériau non tissé, de préférence inférieure à 1 mm.

6. Lingette ou serviette hygiénique jetable dans les toilettes selon l'une quelconque des revendications précédentes, **caractérisée en ce que** les éléments de dissolution (2) sont encastrés par des fibres dans le matériau non tissé.

7. Lingette ou serviette hygiénique jetable dans les toilettes selon l'une quelconque des revendications précédentes, **caractérisée en ce que** les éléments de dissolution (2) ont une couleur ou teinte différente du reste du matériau non tissé.

8. Lingette ou serviette hygiénique jetable dans les toilettes selon l'une quelconque des revendications précédentes, **caractérisée en ce que** les éléments de dissolution ont une forme décorative et/ou d'information, telle que des symboles, des caractères, des logotypes et autres.
